# EUROPEAN PATENT SPECIFICATION

(11) **EP 3 294 909 B1**
(45) Date of publication and mention of the grant of the patent: **17.04.2019**
(21) Application number: 16730486.4
(22) Date of filing: 10.05.2016
(51) Int. Cl.: C12Q 1/6893

(54) **METHOD OF DETECTION AND/OR IDENTIFICATION OF PROTOZOA OF GENUS BABESIA, PRIMER FOR USE IN THIS METHOD AND KIT FOR USE IN METHODS OF DIAGNOSIS OF SYMPTOMATIC AND ASYMPTOMATIC BABESIOSIS**
VERFAHREN ZUR DETEKTION UND/ODER IDENTIFIZIERUNG VON PROTOZOEN DER GATTUNG BABESIA, PRIMER ZUR VERWENDUNG IN DIESEM VERFAHREN UND KIT ZUR VERWENDUNG IN VERFAHREN ZUR DIAGNOSE VON SYMPTOMATISCHER UND ASYMPTOMATISCHER BABESIOSE
PROCÉDÉ DE DÉTECTION ET/OU D'IDENTIFICATION DE PROTOZOAIRES DU GENRE BABESIA, AMORCE UTILE DANS CE PROCÉDÉ ET KIT UTILE DANS DES PROCÉDÉS DE DIAGNOSTIC DE LA BABÉSIOSE SYMPTOMATIQUE ET ASYMPTOMATIQUE

(30) Priority: 12.05.2015 PL 41230915
(43) Date of publication of application: 21.03.2018
(73) Proprietor: Narodowy Instytut Zdrowia Publicznego - Panstwowy Zaklad Higieny, 00-791 Warszawa (PL)
(72) Inventor: ROZEJ-BIELICKA, Wioletta, 05-825 Ksiazenice (PL); MASNY, Aleksander, 04-091 Warszawa (PL); GOLAB, Elzbieta, 05-805 Kanie (PL)
(74) Representative: Grzelak, Anna
(86) International application number: PCT/IB2016/052655
(87) International publication number: WO 2016/181297

(56) References cited:
- WO-A1-2014/197607
- US-A1- 2012 015 360
- WÓJCIK-FATLA ANGELINA ET AL: "Babesia spp. in questing ticks from eastern Poland: prevalence and species diversity.", PARASITOLOGY RESEARCH, vol. 114, no. 8, August 2015 (2015-08), pages 3111-3116, XP002760811, ISSN: 1432-1955

## Description

### Technical field

The present invention relates to a method of detection and/or identification of protozoa of the *Babesia* genus, including in particular *B. microti, B. venatorum, B. canis* and/or *B. divergens* in samples containing nucleic acids, particularly in a genetic material isolated form humans and animals, particularly using nucleic acid amplification techniques, especially PCR and Real-time PCR, especially in a single-tube format. The object of the invention is also a primer for use in the method of detection and/or identification of protozoa of the *Babesia* genus and a kit for use in methods of diagnosis of a *Babesia* infection or a *Babesia* carrier state, for detection and/or identification of protozoa of the *Babesia* genus.

### Background art

*Babesia* are small protozoan parasites of red blood cells of humans and animals. The disease caused by *Babesia* infection, called babesiosis (or more broadly piroplasmosis), is a serious medical and veterinary problem. To date, over 100 distinct species or strains of these protozoa were found and described worldwide. Human cases are caused by several distinct *Babesia* species, for which the most frequently registered causative agents of the infections were: *B. microti, B. divergens* and *B. venatorum* - the latter one also known as sp. EU1 (doi: 10.1016/j.ijppaw.2012.11.003). Whereas, in dogs the disease is caused by 3 *Babesia* species, and in Poland most frequently by *B. canis* (doi: 10.16/j.vetpar.2011.04.023).

Protozoa of the *Babesia* genus are organisms which require two distinct hosts in their life cycle; an intermediate host - animals and humans, and a definitive host - a tick, most frequently Ixodidae. The infection with a particular *Babesia* species depends on the geographic region, which is related to the occurrence of appropriate vectors and intermediate hosts (doi: 10.1186/1471-2334-13-99). The epidemiological data indicate geographic expansion and constant growth of the number of babesiosis cases. *Babesia* infections spreading through a bloodstream route by transfusions (Kogut et al. Emerg Infect Dis. 2005;11(3):476-8) and organ transplantations (Slovut et al. Transplantation. 1996;62(4):537-9) are a growing problem. Several cases of innate babesiosis were also described (Yeruham et al. J Vet Med B Infect Dis Vet Public Health. 2003;50(2):60-2).

The basis of diagnostics of the infections caused by protozoa of the *Babesia* genus are microscopic examinations of blood smears: thick smear and thin smear. Due to the limitations of microscopic methods in diagnostics of infections caused by protozoa of the *Babesia* genus, additional techniques of laboratory examinations are applied, such as serological examination of IgG and/or IgM specific antibodies occurrence by means of: indirect immunofluorescence (IFA), ELISA, immunobloting, immunochromatography, those methods however also having certain limitations. The majority of currently commercially available IFA and ELISA assays is adapted for detection of infections caused by *B. microti,* and do not detect infections caused by the strains dominating in Europe i.e. *B. divergens, B. divergens-like* and *B. venatorum.* In patients with bacterial and viral infections, autoimmune, connective tissue diseases or malaria, false positive results occur frequently (doi: 10.1115/2009/984568). Serological tests are not recommended as the sole test in babesiosis diagnostics, nor for detection of very low levels of antibodies, since those assays may give false negative results in tests of early stages of the disease, in patients after splenectomy, with immunosupression or suffering from AIDS (Schaarschmidt et al. Schweiz Arch Tierheilkd. 2006;148(12):633-40).

In reference laboratories, for detection of *Babesia* infections, polymerase chain reaction (PCR) is used which is approx. 10 times more sensitive in detection of the acute phase of babesiosis than microscopic examination of blood smears (doi: 10.1128/JCM.05848-11). On the market, PCR and Real-time PCR assays are available, which were designed mainly to meet the needs of veterinary diagnostic laboratories(Espy et al. Clin Microbiol Rev. 2006;19(1):165-256) but they are usually specific for a selected strain or genotype (Persing et al. J Clin Microbiol. 1992;30(8):2097-103; Kim et al. Am J Trop Med Hyg. 2007;77(5):837-41), they have limitations of use in environmental studies (Herwaldt et al. Emerg Infect Dis. 2003;9(8):942-8), require sequencing or require additional digestion with restriction enzymes for determination of the *Babesia species* (Bonnet et al. Parasitology. 2007;134(2):197-207).

### Disclosure of invention

The essence of the invention is detection and/or identification and/or differentiation of protozoa of the *Babesia* genus, in single reaction or in a series of separate reactions, based on amplification of nucleic acid with the use of specific primers, designed based on the sequence of *Babesia* genes encoding small ribosomal subunit (18S rRNA) deposited in the GenBank database (http://www.ncbi.nlm.nih.gov): using new primers according to the invention i.e.:
sense primer **Primer 1** (TGACCTAAACCCTCACCAGAGTAAC), SEQ ID NO: 1,
sense primer **Primer 2** (GAATCTAAACCCTTCCCAGAGTATC), SEQ ID NO: 2,
antisense primer **Primer 3** (GCTTTCGCAGTAGTTCGTCTTTA), SEQ ID NO: 3.

The identification numbers of the input sequences (GenBank ID), positions of the primers and the size of the obtained amplicon, along with their use are presented in summary below, in Table 1:

**Table 1: Primers for detection and differentiation of protozoa of the Babesia genus.**

| *Pathogen species* | Primer position | | | Size of the obtained |
|---|---|---|---|---|
| GenBank ID | Primer 1 | Primer 2 | Primer 3 | amplicon (bp) |
| *B. divergens* | 35-59 | - | 393-415 | 381 |
| AY144688 | | | | |
| *B. microti* | - | 21-45 | 406-428 | 407 |
| JQ711225 | | | | |
| *B. venatorum* | 22-46 | - | 380-402 | 381 |
| JX040642 | | | | |
| *B. canis* | 481-503 | - | 839-861 | 381 |
| AY072926 | | | | |

It was unexpectedly found that it is possible to detect and/or identify, preferably in real time, protozoa of many distinct species of the *Babesia* genus, including: *B. microti, B. venatorum* (=*Babesia* sp. EU1), *B. canis, B. divergens,* in samples containing nucleic acids, with the method according to the invention involving a single reaction of amplification of nucleic acids using only two, more preferably three primers designed according to the invention: Primer 1, Primer 2, Primer 3. As was determined by the inventors, due to the risk of contamination with a genetic material of: *Plasmodium, Toxoplasma* or the host, in order to achieve the result, which is to provide the ability to detect and/or identify many distinct species of the *Babesia* genus, using a single reaction of amplification of nucleic acids, the use of primers hybridizing precisely in the sites indicated in Table 1 is necessary. The inventors have shown that moving the primer binding site even by a single position results in decrease of efficiency of the reaction, e.g. moving Primer 3 by a single nucleotide in 3' direction reduces stability of the 3' end, leads to generation of interactions of the primer-dimer type (ΔG -6.09 kcal/mol) and also to hairpin structure formation (the so called hairpin, ΔG -1.59 kcal/mol), thereby impeding proper interactions between the primers and the DNA template. The method of the invention enables detection and/or identification of protozoa of many different species of the *Babesia* genus for diagnosis of symptomatic babesiosis as well as asymptomatic babesiosis.
The object of the invention is thus a method of detection and/or identification of protozoa of the *Babesia* genus, including in particular *B. microti, B. venatorum, B. canis* and/or *B. divergens,* in samples containing nucleic acids, involving the following steps
a) amplification of nucleic acids in one reaction, using primers: Primer 1 of sequence SEQ ID NO: 1 and/or Primer 2 of sequence SEQ ID NO: 2 and Primer 3 of sequence SEQ ID NO: 3;
b) detection and optionally
c) identification.

The detection is conducted with the aim to ascertain that the amplification of the nucleic acids occurred or did not occur in the particular sample containing nucleic acids and with the aim to analyze properties, particularly physical, of the obtained products of amplification.

Identification is conducted based on comparative analysis of the data, for the amplified or unamplified nucleic acids, obtained for the study samples with the results obtained for the amplified or unamplified nucleic acids from the control sample (particularly the positive control sample), adjusted to the particular detection method.

Preferably, the identification is conducted based on comparative analysis of the data obtained from the study samples and the standardized results obtained from the control samples for distinct species of control DNA, particularly for *B. microti* or *B. venatorum* or *B. canis,* or *B. divergens.* This allows to accelerate the process of detection and/or identification of protozoa of the *Babesia* genus, allows to obtain reliable results and leads to prudent use of reagents hence having an economical aspect.

Preferably, the identification is conducted based on comparative analysis of curves obtained for an unknown sample and the curve obtained for the control sample containing for example 10 or 25 or 50 ng/µl of control DNA of *B. microti* or *B. venatorum* or *B. canis,* or *B. divergens* by means of analysis of melting curves in high resolution (HRM) of the obtained amplification products.

In a preferred embodiment, all three primers are used simultaneously, Primer 1 of sequence SEQ ID NO: 1, Primer 2 of sequence SEQ ID NO: 2 and Primer 3 of sequence SEQ ID NO: 3.

In another embodiment, primers Primer 1 of sequence SEQ ID NO: 1 and Primer 3 of sequence SEQ ID NO: 3 are used.

In another embodiment, Primer 2 of sequence SEQ ID NO: 2 and Primer 3 of sequence SEQ ID NO: 3 are used.

In yet another embodiment, apart from primers Primer 1 of sequence SEQ ID NO: 1, Primer 2 of sequence SEQ ID NO: 2 and Primer 3 of sequence SEQ ID NO: 3 one or more other primers are additionally used.

Preferably, at the nucleic acid amplification step, primer hibridization is conducted in temperature in the range of 55-63°C

Preferably, the nucleic acid amplification step is performed using a classical PCR method, multiplex PCR, Real-time PCR.

Preferably in the method of detection and/or identification of protozoa of the *Babesia* genus, the detection step is performed using techniques selected from analysis of physical properties of the obtained amplification products, preferably the analysis of melting curves, of the obtained products, in high resolution (HRM), techniques of detection using molecular probes, amplification product sequencing, analysis of the size and/or mass of the amplification products, differences in migration of conformers of the amplification products, amplification and/or analysis of restriction site polymorphisms.

Preferably, in the method of detection and/or identification of protozoa of the *Babesia* genus, the identification step is performed based on comparative analysis of the data obtained for study samples and the results obtained for control samples adjusted to a particular detection method.

The most preferably in the method of detection and/or identification of protozoa of the *Babesia* genus, both the amplification step and the detection step are performed in a single vessel ("single-tube").

In a particularly preferable embodiment of the method of detection and/or identification of protozoa of the *Babesia* genus, amplification of nucleic acids is performed by a Real-time PCR method and the detection step is conducted using a melting curve analysis of the amplification products, performed in high resolution (HRM), with simultaneous identification step.

Preferably, in the method of detection and/or identification of protozoa of the *Babesia* genus, samples containing nucleic acids were obtained from tissues of humans and/or animals, including arachnids, preferably from blood of humans and/or animals.

The method of detection and/or identification of protozoa according to the invention allows to detect genetic material of many species of the *Babesia* genus (including *B. canis, B. divergens, B. microti, B. venatorum*) using various methods based on PCR. In described in literature and commercially available tests, detecting protozoa of the *Babesia* genus based on nucleic acids amplification techniques, the primers employed in the reaction strictly define the type of PCR in which they can be used. The method of *Babesia* DNA amplification according to the invention using specific primers according to the invention, is characterized by versatility, which means using the primers: Primer 1 and/or Primer 2 and Primer 3 is possible in independent reactions or in the form of a kit for multiplex PCR. The method according to the invention allows use of the same set of primers according to the invention in several types of PCR: classical PCR, multiplex PCR, Real-time PCR. Preferably the reaction is performed in temperature for primer hybridization within the range of 55-63°C. Primer hybridization can also be performed in other temperature ranges.

In the method according to the invention amplicons are obtained, which differ in size from approx. 380 to approx. 410 base pairs, and/or in nucleotide sequence, depending on the species of *Babesia.* Preferably, physical properties of the amplification products are investigated, particularly melting temperature, using melting curve analysis.

The solution according to the invention allows for an easier than in case of prior art methods identification and/or detection of genotype of *Babesia* based on the differences in physical properties of PCR products, particularly shown in the form of DNA melting curves, in preferred embodiment in a "single-tube" format (amplification, detection and identification in single reaction vessel, in one reaction).

The method of detection and/or identification of protozoa according to the invention also allows to identify genetic material of the protozoa of the *Babesia* genus by sequencing amplification products.

The method according to the invention is applicable to medical diagnostics, veterinary diagnostics, epidemiological investigations and environmental investigations.

The method of detection and/or identification of protozoa according to the invention reduces time of conducting detection and/or identification of protozoa of the *Babesia* genus. In a preferred embodiment of the method according to the invention the risk of contamination of the study nucleic acid sample is reduced (reaction of a "single-tube" type).

The method of detection and/or identification of protozoa according to the invention allows to reduce costs of the study, since it decreases the consumption of reagents, does not require sequencing, does not require application of restriction enzymes.

The method according to the invention allows to detect genetic variants of *Babesia* not hitherto described.

The method according to the invention can be adapted to create a commercial diagnostic test for medical laboratories. Such test will have applicability in laboratories, for identification of babesiosis cases, it will also be useful for examinations of blood donors and organ donors for asymptomatic *Babesia* infections.

The method according to the invention can be adapted to create a commercial diagnostic test for veterinary laboratories.

The method according to the invention can be adapted to create a commercial diagnostic test which will be applicable in epidemiological and/or environmental investigations.

The method of detection and/or identification of protozoa according to the invention, in contrast to the previously known methods allows to detect infections with the species dominating in Europe i.e. *B. divergens, B. divergens*-like and *B. venatorum* and is not limited to a selected strain or genotype.

Use of the method of detection and/or identification of protozoa according to the invention allows to avoid false positive results and as a result a correctly diagnosed individual avoids unnecessary and expensive treatment.

Use of the method of detection and/or identification of protozoa according to the invention allows to avoid false negative results and as a result infected correctly diagnosed individual is subjected to the required treatment.

Use of the method of detection and/or identification of protozoa according to the invention due to the wide spectrum of the species of protozoa of the *Babesia* genus identified thereby, is used for diagnosis of both symptomatic and asymptomatic infections caused by protozoa of the *Babesia* genus. Due to the wide identified spectrum of the species of protozoa of the *Babesia* genus the method of detection and/or identification of protozoa according to the invention is preferably used for diagnosis of symptomatic and asymptomatic infections caused by protozoa of the *Babesia* genus since it provides fast and reliable confirmation or exclusion of the infection, and as a result for undertaking appropriate therapeutic or preventive actions.

The present invention also relates to a primer for use in the method of detection and/or identification of protozoa of the *Babesia* genus, in particular including *B. microti, B. venatorum, B. canis* and/or *B. divergens,* in samples containing nucleic acids according to the invention, the primer having the sequence of SEQ ID NO: 1 or SEQ ID NO: 2 or SEQ ID NO: 3.

The present invention also relates to a kit for use in the methods of diagnosis of *Babesia* infections and babesiosis carrier state, used to detect and/or identify protozoa of the *Babesia* genus, including in particular: *B. microti, B. venatorum, B. divergens* and/or *B. canis* in samples containing nucleic acids, the kit including Primer 1 of sequence SEQ ID NO: 1 and/or Primer 2 of sequence SEQ ID NO: 2 and/or Primer 3 of sequence SEQ ID NO: 3 and preferably instructions for use and preferably reagents for PCR.

The present invention also relates to use of the primer Primer 1 of sequence SEQ ID NO: 1 and/or Primer 2 of sequence SEQ ID NO: 2 and Primer 3 of sequence SEQ ID NO: 3 for detection and/or identification of protozoa of the *Babesia* genus, including in particular: *B. microti, B. venatorum, B. canis* and/or *B. divergens,* in samples containing nucleic acids, preferably in samples obtained from tissues of humans and/or animals, including arachnids, preferably from blood of humans and/or animals.

The kit according to the invention preferably simultaneously contains all three primers Primer 1 of sequence SEQ ID NO: 1, Primer 2 of sequence SEQ ID NO: 2 and Primer 3 of sequence SEQ ID NO: 3.

### Brief description of drawings

The invention is explained in more detail in the examples which are however non-limiting to the scope thereof. Embodiments are illustrated on figures, wherein:

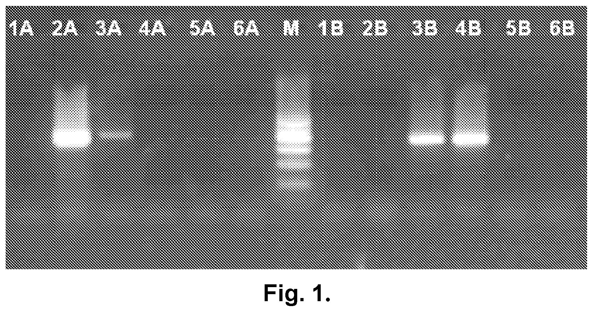

fig. 1. shows an exemplary electrophoretic separation of PCR products, performed on 2% agarose gel stained with ethidium bromide, in two experimental setups (A) left side: Primer 1 + Primer 3 and (B) right side: Primer 2 + Primer 3. Test samples were marked from 1 to 6, M (marker) - mass standard (GBP 600bp DNA Ladder, GenoPlast).

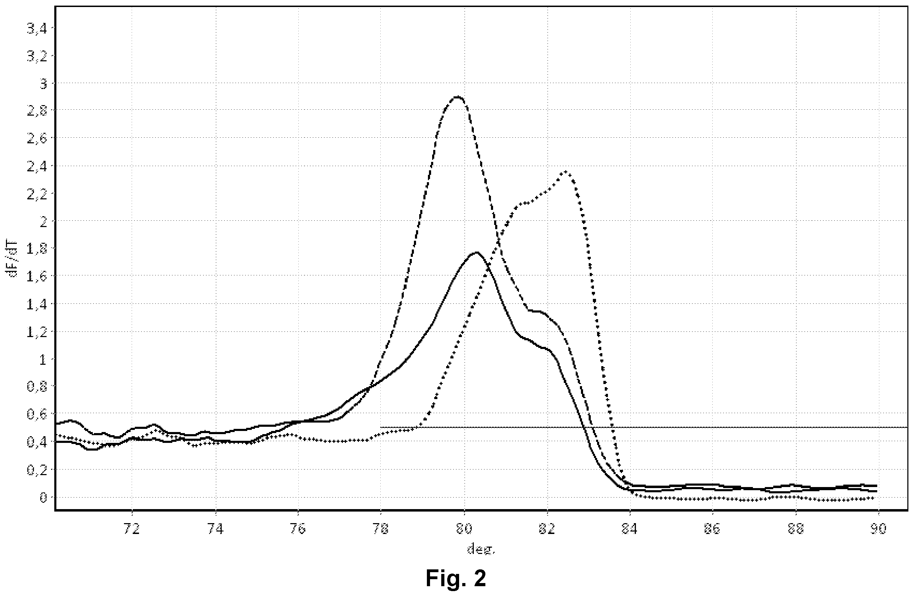

fig. 2. shows DNA melting curves of Real-time PCR products, obtained using primers: Primer 1 + Primer 2 + Primer 3, for samples containing *Babesia* DNA: *B. canis* (solid line), B. *divergens* (-----) and *B. microti* (......).

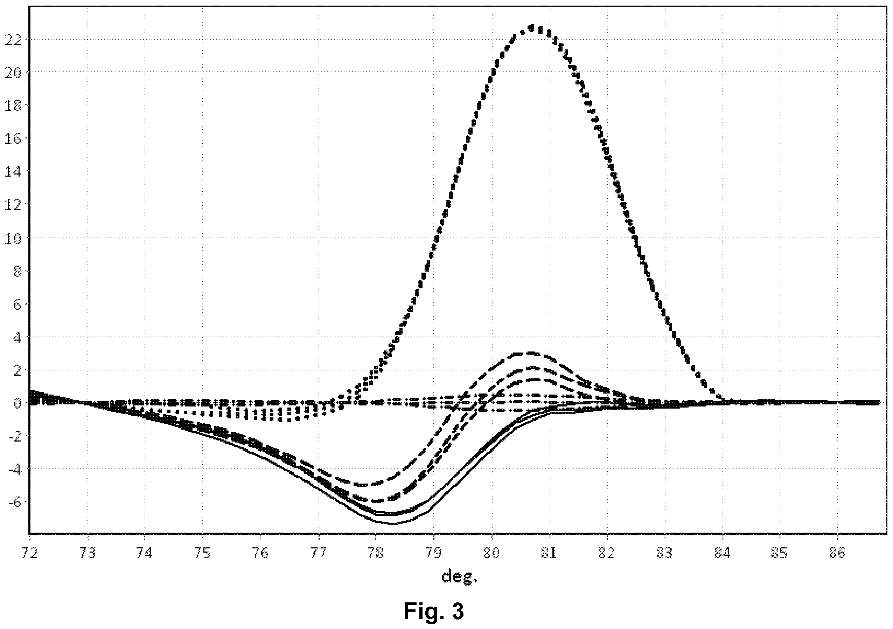

fig. 3. shows an analysis of melting curves of the products obtained in Real-time PCR in comparison to the melting curve of reference DNA (DNA of *B. venatorum*). The results were presented for the samples containing *Babesia* control DNA: *B. canis* (solid line), *B. divergens* (-----) and *B. microti* (......), *B. venatorum* = *Babesia* sp EU1 (-·-·-).

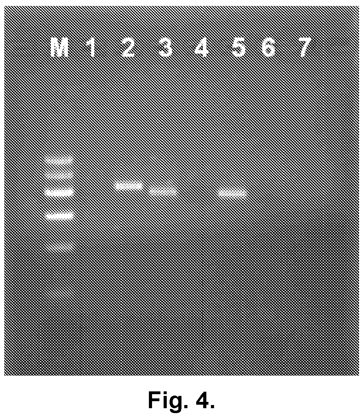

fig. 4. shows results of electrophoretic separation of the products of multiplex Real-time PCR performed using three primers: Primer 1 + Primer 2 + Primer 3, on 4% agarose gel stained with ethidium bromide.

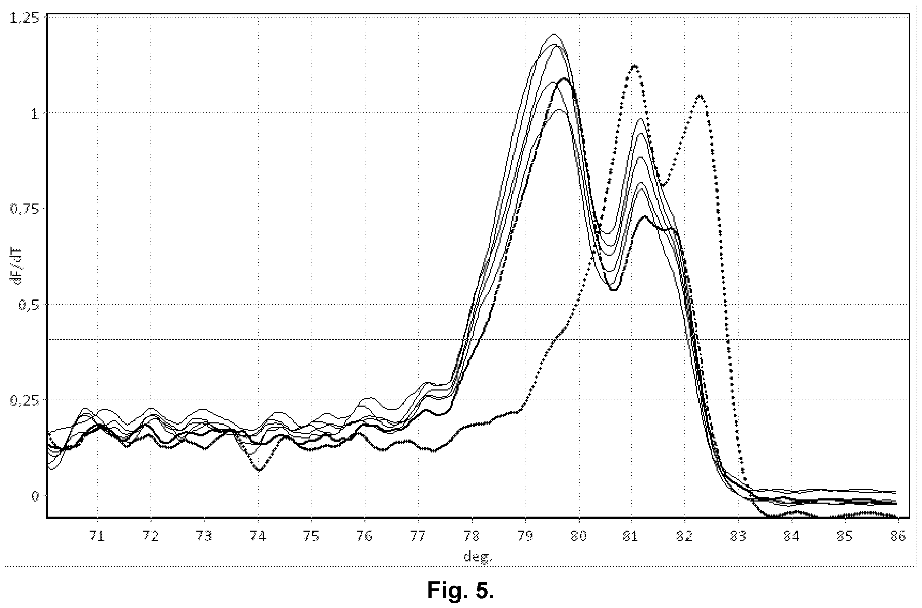

fig. 5. shows results of the analysis of melting of DNA obtained in Real-time PCR using primers: Primer 1, Primer 2, Primer 3 for DNA samples isolated from blood of dogs with babesiosis and two positive controls containing DNA of *B. microti* (......) and *B. canis* (------). Study samples were marked with solid line.

### Description of embodiments

In order to simplify in the description and examples the following abbreviations are used:
18S rRNA - small ribosomal subunit
DNA - deoxyribonucleic acid
HRM - analysis of DNA melting curves in high resolution
multiplex PCR - a method allowing amplification of multiple templates during single PCR reaction.
PCR - polymerase chain reaction
bp - base pairs
Real-time PCR - PCR with real time analysis

### Example 1: Use of primers Primer 1, Primer 2, Primer 3 for Babesia DNA detection by PCR method

The experiment was performed on genetic material isolated from: blood of a root vole naturally infected with *B. microti* (samples marked 2A and 2B), spleen of a deer naturally infected with *B. divergens* (3A and 3B), blood of a dog infected with *B. canis* (4A and 4B). Negative controls: suspension of culture of protozoa *T. gondii* (5A and 5B), human amniotic fluid (6A and 6B), deionized water (1A and 1B).

In the reactions primers of the following sequences were used:

| Primer | sequence (5'→3') | Sequence ID |
|---|---|---|
| Primer 1 | TGACCTAAACCCTCACCAGAGTAAC | SEQ ID NO: 1 |
| Primer 2 | GAATCTAAACCCTTCCCAGAGTATC | SEQ ID NO: 2 |
| Primer 3 | GCTTTCGCAGTAGTTCGTCTTTA | SEQ ID NO: 3 |

PCR was performed in two independent experimental setups, in the following combinations: (part A) Primer 1 + Primer 3 and (part B) Primer 2 + Primer 3. In both cases the reaction was performed in mixture of total volume of 25 µl in the following conditions:

| Components | Volume added (µl/reaction) |
|---|---|
| H₂O | 12.375 |
| Q buffer | 5 |
| PCR buffer | 2.5 |
| dNTPs | 0.5 |
| Primer 1, 10 µM /or Primer 2, 10 µM | 1.25 |
| Primer 3, 10 µM | 1.25 |
| Polymerase Hot Star Taq® DNA | 0.125 |
| Isolate containing genetic material (concentration of total DNA in the sample: 10-500ng/1µl) | 2 |

The reaction took place in polypropylene DNAase and Rnase free tubes of 200 µl volume , for use in PCR. The reaction was performed in 40 cycles according to the temperature profile below:

| Step | | Conditions |
|---|---|---|
| preincubation | | 95°C, 15min |
| amplification | denaturation | 95°C, 60s |
| | annealing | 60°C, 90s |
| | synthesis | 72°C, 60s |
| Final step PCR | | 72°C, 10min |
| Cooling down of reaction mixture | | 8°C |

After reaction completion the obtained PCR products (for *B. microti* - 407 bp, for *B. divergens* and *B. canis* - 381bp) were electrophoretically separated on 2% agarose gel with ethidium bromide added.

Fig. 1 depicts the results of the experiment. The band confirming the presence of parasite's DNA was observed in two wells: 2A - a sample containing DNA from root vole blood naturally infected with *B. microti;* 3A and 3B - samples containing DNA from the spleen of a deer naturally infected with *B. divergens* and DNA from blood of a dog naturally infected with *B. canis* (well 4B). In none of the two investigated experimental setups specific amplification products were observed in negative control samples: *T. gondii* (5A and 5B), human DNA (6A and 6B), deionized water (1A and 1B).

The obtained results indicate applicability of the used amplification method for detection of DNA of protozoa of the *Babesia* species: *B. microti, B. divergens, B. canis* in DNA isolates from human and animal tissues.

### Example 2. Investigation of the specificity of DNA amplification in touch-down PCR using the designed primers Primer 1, Primer 2, Primer 3.

The amplification was performed in the form of touch down PCR in the temperature range from 63°C to 55°C. In the experiment the following primers were used: Primer 1, Primer 2, Primer 3 of sequences described earlier in the *Example 1.* The samples containing the following genetic material were analyzed:
1 - DNA of *Toxoplasma gondii* (*Apicomplexa*)
2 - DNA of *B. microti*
3 - DNA of *B. divergens*
4 - deionized water (H₂O)
5 - DNA of *B.canis*
6 - human DNA
7 - DNA of *I. ricinus* tick

Real Time-PCR was performed in reaction mixture in total volume of 20 µl with use of reagents of the SsoFast™ EvaGreen® Supermix kit, in the following proportions:

| Components | Volume added (µl/reaction) |
|---|---|
| 2X Master Mix | 10 |
| Primer 1, 10 µM | 0.8 |
| Primer 2, 10 µM | 0.8 |
| Primer 3, 10 µM | 0.8 |
| H₂O | 6.6 |
| *Babesia* DNA template (2-200ng/1 µl) | 1 |

The reaction took place in polypropylene (PP) DNAase and Rnase free tubes with increased transparency, of 200 µl volume, for use in PCR. The amplification was performed in 45 cycles with reduction of hybridization temperature by 0.2°C in each cycle in the following conditions:

| cycle | conditions |
|---|---|
| preincubation | Hold @ 98°C, 2min, 0s |
| amplification | Step 1 @ 98°C, hold 10s |
| | Step 2 @ 63°C, hold 30s |
| | Step 3 @ 72°C, hold 30s, acquiring to Cycling A ([Green]) |
| Melting analysis | Melt (70-90°C), hold secs on the 1st step, hold 3 secs on next steps, Melt A ([HRM]) |

The melting curves obtained as a result of the performed experiment are shown on the figure (fig. 2). The results of the experiment performed according to the invention may be analyzed in a normalized form, which was shown on fig. 3. After completion of the reaction, the obtained amplicons (for *B. microti* - 407bp, *B. divergens* and *B. canis* - 381bp) were visualized after performing electrophoresis in agarose gel with addition of ethidium bromide using 4% agarose (Prona) allowing to observe the differences between the obtained amplification products. The photograph of the gel is shown on fig. 4.

The obtained results indicate applicability of the used amplification method for detection and differentiation of Babesia protozoa DNA of species: *B. microti, B. divergens, B. canis* in clinical and environmental samples.

### Example 3. Detection and identification of Babesia DNA in genetic material isolated from blood samples collected from 5 dogs with clinically recognized babesiosis confirmed by microscopic examination.

The study samples contained DNA isolated from blood of dogs with confirmed babesiosis. The reaction was performed in total volume of 25 µl using the same set of reagents as in Example 2, in the following conditions:

| Components | Volume added (µl/reaction) |
|---|---|
| 2X Master Mix | 12.5 |
| Primer 1, 10 µM | 1 |
| Primer 2, 10 µM | 1 |
| Primer 3, 10 µM | 1 |
| H₂O | 8.5 |
| DNA template (total DNA 50-100ng/1 µl) | 1 |

The reaction was performed in the conditions described earlier in Example 2. The results of the reaction are shown on fig. 5 wherein: samples containing DNA isolated from blood of 5 dogs with babesiosis are marked with solid line, positive control containing *B. microti* DNA is shown in dotted line (.....), and *B. canis* - dashed line (---). Comparative analysis of the curves obtained for the study samples and the control curves of the samples containing reference DNA of *Babesia* indicates that the etiological agent of the detected infections was *B. canis* species.

The obtained results indicate applicability of the used amplification method for detection and differentiation of DNA of protozoa *B. microti* and *B. canis* in clinical samples.

## Claims

1. A method of detection and/or identification of protozoa of the *Babesia* genus including, in particular *B. microti, B. venatorum, B. canis* and/or *B. divergens* in samples containing nucleic acids **characterized in that** the method comprises the following steps:
a) amplification of nucleic acids in a single reaction, using primers: Primer 1 of sequence consisting of SEQ ID NO: 1 and/or Primer 2 of sequence consisting of SEQ ID NO:2 and Primer 3 of sequence consiting of SEQ ID NO:3;
b) detection; and optionally
c) identification.

2. The method according to the claim 1, **characterized in that** all three primers Primer 1 of sequence SEQ ID NO: 1, Primer 2 of sequence SEQ ID NO:2 and Primer 3 of sequence SEQ ID NO:3 are used simultaneously.

3. The method according to the claim 1, **characterized in that**
a) primers Primer 1 of sequence SEQ ID NO: 1 and Primer 3 of sequence SEQ ID NO:3 are used, or
b) primers Primer 2 of sequence SEQ ID NO: 2 and Primer 3 of sequence SEQ ID NO:3 are used.

4. The method according to the claim 1-3, **characterized in that** one or more other primers are additionally used.

5. The method according to any of claims 1-4, **characterized in that** at the nucleic acids amplification step primer hybridization is performed in the temperature range of 55-63°C.

6. The method according to any of claims 1-5, **characterized in that** the nucleic acids amplification step is performed by a method of classical PCR, multiplex PCR, Real-time PCR.

7. The method according to any of claims 1-6, **characterized in that** the detection step is performed with techniques selected from analysis of physical properties of the obtained amplification products, preferably melting curve analysis for the obtained amplification products, in high resolution (HRM), detection techniques using molecular probes, amplification product sequencing, size and/or mass analysis of the amplification products, differences in migration of conformers of the amplification products, amplification and or analysis of restriction site polymorphism.

8. The method according to any of claims 1-7, **characterized in that** the identification step is performed based on comparative analysis of the data obtained for study samples and results obtained for controls suitable for a particular detection method.

9. The method according to any of claims 1-8, **characterized in that** both the nucleic acids amplification and detection steps are performed in one vessel ("single-tube").

10. The method according to any of claims 1-9, **characterized in that** the nucleic acids amplification step is performed by a Real-time PCR method and the detection step is performed by a melting curve analysis method for the obtained amplification products, in high resolution with a simultaneous identification step.

11. The method according to any of claims 1-10, **characterized in that** the samples containing nucleic acids were obtained from tissues of humans and/or animals, including arachnids, preferably from blood of humans and/or animals.

12. A primer for use in a method of detection and/or identification of protozoa of the *Babesia* genus, including in particular *B. microti, B. venatorum, B. canis* and/or *B. divergens* in samples containing nucleic acids as defined in any of claims 1-11, **characterized in that** it has the sequence consisiting of SEQ ID NO: 1 or the sequence consisting of SEQ ID NO:2 or the sequence consisting of SEQ ID NO:3.

13. Use of Primer 1 of sequence consisting of SEQ ID NO: 1 and/or Primer 2 of sequence consisting of SEQ ID NO:2 and Primer 3 of sequence consisting of SEQ ID NO:3 in the method of detection and/or identification of protozoa of the *Babesia* genus, including in particular *B. microti, B. venatorum, B. canis* and/or *B. divergens* in samples containing nucleic acids, preferably in samples obtained from tissues of human and animals, including arachnids, preferably from blood of humans and/or animals, wherein the method of detection and/or identification of protozoa of the *Babesia* genus comprises amplification of nucleic acids, using primers: Primer 1 of sequence consisting of SEQ ID NO: 1 and/or Primer 2 of sequence consisting of SEQ ID NO:2 and Primer 3 of sequence consisting of SEQ ID NO:3, and wherein the nucleic acids amplification step is performed by a method of classical PCR, multiplex PCR, Real-time PCR.

14. A kit for use in methods of diagnosis of a *Babesia* infection or a *Babesia* carrier state, including detection and/or identification of protozoa of the *Babesia* genus, including in particular *B. microti, B. venatorum, B. canis* and/or *B. divergens,* in samples containing nucleic acids, **characterized in that** it comprises Primer 1 of sequence consisting of SEQ ID NO: 1 and/or Primer 2 of sequence consisting of SEQ ID NO:2 and Primer 3 of sequence consisting of SEQ ID NO:3 and preferably, instructions for use and preferably reagents for PCR.

15. The kit according to the claim 14, **characterized in that** it contains simultaneously all three primers Primer 1 of sequence SEQ ID NO: 1, Primer 2 of sequence SEQ ID NO:2 and Primer 3 of sequence SEQ ID NO:3.

## Patentansprüche

1. Verfahren zum Nachweis und/oder zur Identifizierung von Protozoen der Gattung *Babesia* umfassend insbesondere *B. microti, B. venatorum, B. canis* und/oder *B. divergens* in Proben, die Nukleinsäuren enthalten, **dadurch gekennzeichnet, dass** das Verfahren die folgenden Schritte umfasst:
a) Amplifikation von Nukleinsäuren in einer einzigen Reaktion unter Verwendung von Primern: Primer 1 mit der Sequenz zusammensetzt aus SEQ ID NO: 1 und/oder Primer 2 mit der Sequenz zusammensetzt aus SEQ ID NO: 2 und Primer 3 mit der Sequenz zusammensetzt aus SEQ ID NO: 3;
b) Nachweis; und optional
c) Identifizierung.

2. Verfahren nach Anspruch 1, **dadurch gekennzeichnet, dass** alle drei Primer Primer 1 mit der Sequenz SEQ ID NO: 1, Primer 2 mit der Sequenz SEQ ID NO: 2 und Primer 3 mit der Sequenz SEQ ID NO: 3 gleichzeitig verwendet werden.

3. Verfahren nach Anspruch 1, **dadurch gekennzeichnet, dass**
a) Primer Primer 1 mit der Sequenz SEQ ID NO: 1 und Primer 3 mit der Sequenz SEQ ID NO: 3 verwendet werden, oder
b) Primer Primer 2 mit der Sequenz SEQ ID NO: 2 und Primer 3 mit der Sequenz SEQ ID NO: 3 verwendet werden.

4. Verfahren nach Anspruch 1-3, **dadurch gekennzeichnet, dass** zusätzlich ein oder mehrere andere Primer verwendet werden.

5. Verfahren nach einem der Ansprüche 1-4, **dadurch gekennzeichnet, dass** in einem Schritt der Nukleinsäureamplifikation die Hybridisierung der Primer im Temperaturbereich von 55-63°C durchgeführt wird

6. Verfahren nach einem der Ansprüche 1-5, **dadurch gekennzeichnet, dass** den Schritt der Nukleinsäureamplifikation durch ein Verfahren der klassischen PCR, Multiplex-PCR, Real-time PCR durchgeführt wird.

7. Verfahren nach einem der Ansprüche 1-6, **dadurch gekennzeichnet, dass** der Nachweisschritt mit Techniken ausgewählt aus der Analyse der physikalischen Eigenschaften der erhaltenen Amplifikationsprodukte, vorzugsweise der Schmelzkurvenanalyse der erhaltenen Amplifikationsprodukte, in hoher Auflösung (HRM), Detektionstechniken unter Verwendung der molekulare Sonden, Sequenzierung der Amplifikationsprodukte, Größen- und/oder Massenanalyse der Amplifikationsprodukte, der Unterschiede in der Migration von Konformeren der Amplifikationsprodukte, Amplifikation und/oder Analyse des Restriktionsstellenpolymorphismus durchgeführt wird.

8. Verfahren nach einem der Ansprüche 1-7, **dadurch gekennzeichnet, dass** der Identifizierungsschritt auf der Grundlage einer vergleichenden Analyse der für die Untersuchungsproben erhaltenen Daten und der Ergebnisse für die für ein bestimmtes Nachweisverfahren geeigneten Kontrollen durchgeführt wird.

9. Verfahren nach einem der Ansprüche 1-8, **dadurch gekennzeichnet, dass** sowohl die Nukleinsäureamplifikations- als auch der Nachweisschritt in einem Gefäß ("Einzelröhrchen") durchgeführt werden.

10. Verfahren nach einem der Ansprüche 1-9, **dadurch gekennzeichnet, dass** der Nukleinsäureamplifikationsschritt durch ein Real-time-PCR-Verfahren durchgeführt wird und der Nachweisschritt durch ein Verfahren der Schmelzkurvenanalyse für die erhaltenen Amplifikationsprodukte in hoher Auflösung mit einem gleichzeitigen Identifizierungsschritt durchgeführt wird.

11. Verfahren nach einem der Ansprüche 1-10, **dadurch gekennzeichnet, dass** die Nukleinsäuren enthaltenden Proben aus Geweben der Menschen und/oder Tieren, einschließlich Spinnentieren, vorzugsweise aus Blut der Menschen und/oder Tieren gewonnen wurden.

12. Primer zur Verwendung in dem Nachweis- und/oder Identifizierungsverfahren von Protozoen der Gattung *Babesia* umfassend insbesondere *B. microti, B. venatorum, B. canis* und/oder *B. divergens* in Proben, die Nukleinsäuren, wie es in einem der Ansprüche 1-11 definiert wird, enthalten, **dadurch gekennzeichnet, dass** es die Sequenz zusammensetzt aus SEQ ID NO: 1 oder die Sequenz zusammensetzt aus SEQ ID NO: 2 oder die Sequenz zusammensetzt aus SEQ ID NO: 3 hat.

13. Verwendung von Primer 1 mit der Sequenz zusammensetzt aus SEQ ID NO: 1 und/oder Primer 2 mit der Sequenz zusammensetzt aus SEQ ID NO: 2 und Primer 3 mit der Sequenz zusammensetzt aus SEQ ID NO: 3 im Verfahren zum Nachweis und/oder zur Identifizierung von Protozoen der Gattung *Babesia,* umfassend insbesondere *B. microti, B. venatorum, B. canis* und/oder *B. divergens* in die Nukleinsäure enthaltenden Proben, vorzugsweise in Proben aus Geweben der Menschen und Tieren, einschließlich Spinnentieren, vorzugsweise aus Blut der Menschen und/oder Tieren, wobei das Nachweis- und/oder Identifizierungsverfahren von Protozoen der Gattung *Babesia* die Amplifikation von Nukleinsäuren unter Verwendung von Primern: Primer 1 mit der Sequenz zusammensetzt aus SEQ ID NO: 1 und/oder Primer 2 mit der Sequenz zusammensetzt aus SEQ ID NO: 2 und Primer 3 mit der Sequenz zusammensetzt aus SEQ ID NO: 3 umfasst, und wobei der Schritt der Nukleinsäureamplifikation mit einem Verfahren der klassischen PCR, Multiplex-PCR, Real-time PCR durchgeführt wird.

14. Kit zur Verwendung in Diagnoseverfahren für eine *Babesi*a-Infektion oder einen *Babesia*-Trägerstatus einschließlich Nachweis und/oder Identifizierung von Protozoen der Gattung *Babesia* umfassend insbesondere *B. microti, B. venatorum, B. canis* und/oder *B. divergens* in Proben, die Nukleinsäuren enthalten, **dadurch gekennzeichnet, dass** es Primer 1 mit der Sequenz zusammensetzt aus SEQ ID NO: 1 und/oder Primer 2 mit der Sequenz zusammensetzt aus SEQ ID NO: 2 und Primer 3 mit der Sequenz zusammensetzt aus SEQ ID NO: 3 und vorzugsweise Gebrauchsanweisungen und vorzugsweise Reagenzien für die PCR beinhaltet.

15. Ein Kit nach Anspruch 14, **dadurch gekennzeichnet, dass** es alle drei Primer Primer 1 der Sequenz SEQ ID NO: 1, Primer 2 der Sequenz SEQ ID NO: 2 und Primer 3 der Sequenz SEQ ID NO: 3 gleichzeitig beinhaltet.

## Revendications

1. Une méthode de détection et/ou d'identification de protozoaires du genre *Babesia,* y compris en particulier *B. microti, B. venatorum, B. canis* et/ou *B. divergens* dans des échantillons contenant des acides nucléiques **caractérisée en ce que** la méthode comprend les étapes suivantes :
a) amplification des acides nucléiques en une seule réaction, en utilisant des amorces : Amorce 1 de séquence constituée de SEQ ID NO : 1 et/ou Amorce 2 de séquence constituée de SEQ ID NO : 2 et Amorce 3 de séquence constituée de SEQ ID NO : 3 ;
b) détection ; et éventuellement
c) identification.

2. La méthode selon la revendication 1, **caractérisée en ce que** tous les trois amorces Amorce 1 de séquence SEQ ID NO : 1, Amorce 2 de séquence SEQ ID NO : 2 et Amorce 3 de séquence SEQ ID NO : 3 sont utilisées simultanément.

3. La méthode selon la revendication 1, **caractérisée en ce que**
a) les amorces Amorce 1 de séquence SEQ ID NO : 1 et Amorce 3 de séquence SEQ ID NO : 3 sont utilisées, ou
b) les amorces Amorce 2 de séquence SEQ ID NO : 2 et Amorce 3 de séquence SEQ ID NO : 3 sont utilisées.

4. La méthode selon les revendications 1-3, **caractérisée en ce qu'**une ou plusieurs autres amorces sont en outre utilisées.

5. La méthode selon l'une quelconque des revendications 1-4, **caractérisée en ce qu'**à l'étape d'amplification des acides nucléiques, l'hybridation d'amorce est effectuée dans la plage de températures de 55-63°C

6. La méthode selon l'une quelconque des revendications 1-5, **caractérisée en ce que** l'étape d'amplification des acides nucléiques est réalisée par une méthode de PCR classique, PCR multiplex, PCR en temps réel.

7. La méthode selon l'une quelconque des revendications 1-6, **caractérisée en ce que** l'étape de détection est réalisée avec des techniques choisies parmi l'analyse des propriétés physiques des produits d'amplification obtenus, de préférence l'analyse de la courbe de fusion pour les produits d'amplification obtenus, en haute résolution (HRM), des techniques de détection utilisant des sondes moléculaires, le séquençage de produits d'amplification, l'analyse de taille et/ou de masse des produits d'amplification, différences de migration des conformères des produits d'amplification, l'amplification et/ou l'analyse du polymorphisme du site de restriction.

8. La méthode selon l'une quelconque des revendications 1-7, **caractérisée en ce que** l'étape d'identification est réalisée sur la base d'une analyse comparative des données obtenues pour des échantillons d'étude et des résultats obtenus pour des contrôles appropriés à un procédé de détection particulier.

9. La méthode selon l'une quelconque des revendications 1-8, **caractérisée en ce que** les étapes d'amplification et de détection des acides nucléiques sont réalisées dans un seul récipient (« single-tube »).

10. La méthode selon l'une quelconque des revendications 1-9, **caractérisée en ce que** l'étape d'amplification des acides nucléiques est réalisée par une méthode de PCR en temps réel et l'étape de détection est réalisée par une méthode d'analyse de la courbe de fusion pour des produits d'amplification obtenus, en haute résolution, avec une étape d'identification simultanée.

11. La méthode selon l'une quelconque des revendications 1-10, **caractérisée en ce que** les échantillons contenant des acides nucléiques ont été obtenus à partir des tissus des humains et/ou animaux, y compris des arachnides, de préférence à partir du sang des humains et/ou animaux.

12. Une amorce à utiliser dans une méthode de détection et/ou d'identification de protozoaires du genre *Babesia,* y compris notamment *B. microti, B. venatorum, B. canis* et/ou *B. divergens* dans des échantillons contenant des acides nucléiques, comme définie dans l'une quelconque des revendications 1-11, **caractérisée en ce qu'**elle a la séquence constituée de SEQ ID NO : 1 ou la séquence constituée de SEQ ID NO : 2 ou la séquence constituée de SEQ ID NO : 3.

13. Utilisation de l'Amorce 1 de séquence constituée de SEQ ID NO : 1 et/ou Amorce 2 de séquence constituée de SEQ ID NO : 2 et Amorce 3 de séquence constituée de SEQ ID NO : 3 dans la méthode de détection et/ou d'identification de protozoaires du genre *Babesia,* y compris en particulier *B. microti, B. venatorum, B. canis* et/ou *B. divergens* dans des échantillons contenant des acides nucléiques, de préférence des échantillons à partir de tissus des humains et des animaux, y compris les arachnides, de préférence à partir du sang des humains et/ou des animaux, dans laquelle la méthode de détection et/ou d'identification de protozoaires du genre *Babesia* comprend l'amplification des acides nucléiques, en utilisant des amorces: Amorce 1 de séquence constituée de SEQ ID NO : 1 et/ou Amorce 2 de séquence constituée de SEQ ID NO : 2 et Amorce 3 de séquence constituée de SEQ ID NO : 3, et dans laquelle l'étape d'amplification des acides nucléiques est réalisée par une méthode de PCR classique, PCR multiplex, PCR en temps réel.

14. Un kit à utiliser dans les méthodes de diagnostic d'une infection *Babesia* ou d'un état de porteur *Babesia,* y compris la détection et/ou l'identification de protozoaires du genre Babesia, y compris en particulier *B. microti, B. venatorum, B. canis* et/ou *B. divergens* dans des échantillons contenant des acides nucléiques, **caractérisé en ce qu'**il comprend Amorce 1 de séquence constituée de SEQ ID NO : 1 et/ou Amorce 2 de séquence constituée de SEQ ID NO : 2 et Amorce 3 de séquence constituée de SEQ ID NO : 3 et de préférence des instructions d'utilisation et de préférence des réactifs pour PCR.

15. Le kit selon la revendication 14, **caractérisé en ce qu'**il contient simultanément tous les trois amorces Amorce 1 de séquence SEQ ID NO : 1, Amorce 2 de séquence SEQ ID NO : 2 et Amorce 3 de séquence SEQ ID NO : 3.


### SEQUENCE LISTING

<110> NIZP_PZH
<120> Method of detection and/or identification of protozoa of genus Babesia, primer for use in this method and kit for use in methodsof diagnosis of symptomatic and asymptomatic babesiosis
<130> PZ/3104/AGR/PCT
<160> 3
<170> PatentIn version 3.5

<210> 1
   <211> 25
   <212> DNA
   <213> artificial
<220>
   <223> Starter 1
<400> 1
   tgacctaaac cctcaccaga gtaac 25
<210> 2
   <211> 25
   <212> DNA
   <213> artificial
<220>
   <223> Starter 2
<400> 2
   gaatctaaac ccttcccaga gtatc 25
<210> 3
   <211> 23
   <212> DNA
   <213> artificial
<220>
   <223> Starter 3
<400> 3
   gctttcgcag tagttcgtct tta 23

### SEQUENCE LISTING

<110> NIZP_PZH
<120> Method of detection and/or identification of protozoa of genus Babesia, primer for use in this method and kit for use in methods of diagnosis of symptomatic and asymptomatic babesiosis
<130> PZ/3104/AGR/PCT
<160> 3
<170> PatentIn version 3.5
<210> 1
   <211> 25
   <212> DNA
   <213> artificial
<220>
   <223> Starter 1
<400> 1
   tgacctaaac cctcaccaga gtaac 25
<210> 2
   <211> 25
   <212> DNA
   <213> artificial
<220>
   <223> Starter 2
<400> 2
   gaatctaaac ccttcccaga gtatc 25
<210> 3
   <211> 23
   <212> DNA
   <213> artificial
<220>
   <223> Starter 3
<400> 3
   gctttcgcag tagttcgtct tta 23